# DEMANDE DE BREVET EUROPEEN

(11) **EP 4 529 948 A1**
(43) Date de publication de la demande: **02.04.2025**
(21) Numéro de dépôt: 24203421.3
(22) Date de dépôt: 29.09.2024
(51) Int. Cl.: A61N 5/06

(54) **DISPOSITIF D'ÉCLAIRAGE CIRCADIEN**

(30) Priorité: 30.09.2023 FR 2310476
(71) Demandeur: Lucibel SA, 76360 Barentin (FR)
(72) Inventeur: DAVID, Thibault, 76240 Bonsecours (FR); HOOPER, Marcel, 76000 Rouen (FR)
(74) Mandataire: Martin, Marie-Aude

(57) **Abrégé**

Le dispositif (20) d'éclairage circadien d'éclairage circadien comprend une source lumineuse (200) accordable pour fournir une pluralité de scénarios d'éclairage prédéterminés. La source (200) comprend une première unité (212) de lumière blanche dont le spectre lumineux s'étend principalement dans une plage de longueurs d'onde comprise entre 400 nm et 800 nm et une deuxième unité (214) de lumière bleue dont le spectre lumineux forme un pic étroit dont le sommet est compris entre 400 et 500 nm. Le dispositif (20) comprend un module de commande (220) de la puissance lumineuse des deux unités (210 ; 212, 214) indépendamment l'une de l'autre afin d'obtenir un ratio de mixage spectral des deux unités, variable en fonction du temps, par la variation de la puissance lumineuse de chacune des unités (210 ; 212, 214) entre des valeurs maximales et minimales, la puissance lumineuse émise par la deuxième unité (214) étant toujours inférieure ou égale à 35% de la puissance totale émise par la source lumineuse (200).

## Description

La présente invention concerne un dispositif d'éclairage à fonction d'éclairage circadien, en particulier pour la synchronisation d'une horloge biologique d'un organisme humain.

L'horloge biologique ou circadienne permet à l'organisme humain de réguler certaines des fonctions du corps humain sur une période d'une journée, plus précisément sur une période pouvant varier entre 23h30 et 24h30 selon les individus.

Située dans le cerveau, cette horloge biologique est composée de plusieurs milliers de neurones dont l'activité pulsatile contrôle notamment le cycle éveil/sommeil, la température corporelle, le rythme cardiaque, la sécrétion d'hormones, la division des cellules, la réparation de l'ADN, etc.

Afin de se resynchroniser en permanence sur une journée, cette horloge prend en compte des signaux de l'environnement extérieur, et notamment la lumière. Une exposition inappropriée à la lumière, par exemple produite par un éclairage artificiel, peut créer un déphase de l'horloge biologique avec des conséquences sur les fonctions cognitives, le sommeil, la vigilance, la mémoire, les fonctions cardiovasculaires de l'organisme humain.

Cette horloge biologique est susceptible d'être déréglée : exposition prolongée à des écrans, décalage horaire, travail de nuit, mauvaise hygiène de vie, etc. Des effets indésirables et désagréables sur le corps humain sont alors ressentis durant plusieurs jours : perturbation du cycle sommeil/éveil, difficultés de concentration, irritabilité, dépression saisonnière.

Par exemple, la traversée d'une pluralité de fuseaux horaires, lors de vols long-courriers, affecte l'horloge interne du corps humain et désynchronise le cycle éveil/sommeil par rapport au lieu d'arrivée. Ce phénomène est connu sous le nom de syndrome du décalage horaire, ou selon l'appellation anglo-saxonne « jetlag ».

En particulier, pour remédier au syndrome du décalage horaire, une solution repose sur l'utilisation de dispositifs de luminothérapie, avant le vol ou après le vol. Ces dispositifs de luminothérapie sont par exemple connus pour le traitement de la dépression saisonnière.

Les dispositifs de luminothérapie existants peuvent être regroupés en deux catégories fonctionnelles : la luminothérapie et l'éclairage à balance variable des blancs, plus connu sous l'expression anglo-saxonne « tunable white ».

La luminothérapie permet de produire ponctuellement un éclairage intense avec un spectre proche de celui du soleil à un moment précis de la journée. Le traitement a généralement une durée inférieure à une heure et présente un spectre fixe et prédéfini par le fabricant.

Avec l'éclairage à balance variable des blancs, le luminaire est configuré pour générer une lumière blanche dont le spectre lumineux varie de façon dynamique typiquement dans des gammes de température de couleur allant de 2700K à 6500K et dont l'intensité lumineuse est également réglable.

Ces dispositifs ont pour objectif principal de stimuler un cycle de lumière naturelle dans un environnement intérieur et reproduisent, soit artificiellement la lumière du soleil sur une durée d'une journée soit de façon plus ponctuelle et intense sur une courte durée, une lumière blanche proche de la lumière du soleil au Zenith.

Afin de remédier au syndrome du décalage horaire, ces dispositifs offrent une approche globale qui consiste à recréer artificiellement la lumière du soleil et soumettre le passager à une alternance de phase d'exposition à la lumière et d'obscurité avant son départ ou à son arrivée. Mais cette solution donne de faibles résultats et est contraignante pour le passager.

L'invention a notamment pour but de proposer des dispositifs d'éclairage à fonction d'éclairage circadien efficace pour synchroniser l'horloge biologique d'un individu.

### Description de l'invention

A cet effet, l'invention a pour objet un dispositif d'éclairage circadien comprenant une source lumineuse accordable pour fournir une pluralité de scénarios d'éclairage prédéterminés, caractérisé en ce que la source comprend une première unité de lumière blanche dont le spectre lumineux s'étend principalement dans une plage de longueurs d'onde comprise entre 400 nm et 800 nm et une deuxième unité de lumière bleue dont le spectre lumineux forme un pic étroit dont le sommet est compris entre 400 et 500 nm et en ce que le dispositif comprend un module de commande de la puissance lumineuse des deux unités indépendamment l'une de l'autre afin d'obtenir un ratio de mixage spectral des deux unités, variable en fonction du temps, par la variation de la puissance lumineuse de chacune des unités entre des valeurs maximales et minimales, la puissance lumineuse émise par la deuxième unité étant toujours inférieure ou égale à 35% de la puissance totale émise par la source lumineuse.

L'efficacité du dispositif de l'invention repose principalement sur l'activation par la deuxième unité des cellules ganglionnaires à mélanopsine situées dans la rétine de l'œil. Ces cellules photoréceptrices sont essentielles à la transmission de l'information lumineuse vers de nombreux centres du cerveau non-visuels. L'activation ou non de cette deuxième unité permet d'accentuer artificiellement la stimulation ou non des voies non visuelles et permet d'influer ainsi notamment sur la sécrétion d'hormones de l'organisme humain soumis à cet éclairage. Le dispositif de l'invention offre ainsi une synchronisation biologique dynamique permettant une personnalisation du traitement en fonction des besoins d'un individu.

Un dispositif selon l'invention peut comprendre l'une ou plusieurs des caractéristiques suivantes.

Dans un mode de réalisation préféré de l'invention, dans un scénario de stimulation, le module de commande est configuré pour faire varier progressivement la puissance lumineuse de la deuxième unité d'une puissance nulle jusqu'à une puissance maximale correspondant à au plus 35% de la puissance totale de la source lumineuse.

Dans un mode de réalisation préféré de l'invention, la première unité de lumière blanche comprend un premier sous-canal de lumière blanc chaud avec une température de couleur autour de 6500 Kelvin et un deuxième sous-canal de lumière blanc froid avec une température de couleur autour de 2700 Kelvin.

Dans un mode de réalisation préféré de l'invention, le module de commande est configuré pour faire varier la puissance lumineuse de la deuxième unité entre une valeur nulle et une valeur maximale selon un profil de trains d'impulsions, par exemple de période de répétition comprise entre 12 minutes et 18 minutes.

Dans un mode de réalisation préféré de l'invention, la source comprend une troisième unité dont le spectre lumineux est relativement enrichi de longueurs d'onde supérieures à 560nm et relativement dépourvu de longueurs d'onde inférieures à 560 nm.

Dans un mode de réalisation préféré de l'invention, dans un scénario de récupération, le module de commande est configuré pour faire varier la puissance lumineuse de la première unité jusqu'à une puissance sensiblement nulle et pour maintenir uniquement la puissance lumineuse de la troisième unité à une valeur non nulle.

Dans un mode de réalisation préféré de l'invention, la durée d'un scénario de traitement est comprise entre 80 minutes et 100 minutes, de préférence 90 minutes.

Dans un mode de réalisation préféré de l'invention, le dispositif comprenant une interface utilisateur, le module de commande comprend un moyen de génération des signaux de pilotage des unités d'éclairage en fonction d'une trame de données générée consécutivement à la sélection par un opérateur d'un scénario parmi une pluralité de scénarios prédéterminés.

Dans un mode de réalisation préféré de l'invention, le dispositif comprend un module de recommandation configuré pour générer un niveau de recommandation pour chaque scénario d'éclairage et un créneau horaire recommandé pour appliquer chaque scénario.

Dans un mode de réalisation préféré de l'invention, ledit module de recommandation comprend un moyen de saisie pour recueillir des données relatives à un utilisateur et un moyen de détermination du niveau de recommandation pour chaque scénario à partir d'une combinaison des données saisies et d'une information d'un créneau horaire actuel local.

Dans un mode de réalisation préféré de l'invention, les données relatives à l'utilisateur comprennent une liste de paramètres choisis parmi un chronotype, une heure de réveil et/ou une heure de coucher et un décalage horaire.

Dans un mode de réalisation préféré de l'invention, le dispositif comprend une interface utilisateur supportée de manière logicielle sur un équipement électronique qui communique à distance avec le module de commande.

Tel qu'il est utilisé dans le présent document, le terme « DEL » ou « LED » doit être compris comme incluant toute diode électroluminescente ou tout autre type de système basé sur l'injection/jonction de porteurs capable de générer un rayonnement en réponse à un signal électrique. Ainsi, le terme LED comprend, sans s'y limiter, diverses structures à base de semi-conducteurs qui émettent de la lumière en réponse à un courant, des polymères émetteurs de lumière, des diodes électroluminescentes organiques (OLED), des bandes électroluminescentes, etc. En particulier, le terme LED fait référence aux diodes électroluminescentes de tous types (y compris les diodes électroluminescentes semi-conductrices et organiques) qui peuvent être configurées pour générer un ou plusieurs rayonnements dans le spectre infrarouge, le spectre ultraviolet et diverses parties du spectre visible (comprenant généralement des longueurs d'onde de rayonnement allant d'environ 400 nanomètres à environ 700 nanomètres). Parmi les exemples de diodes électroluminescentes, on peut citer, sans s'y limiter, divers types de diodes infrarouges, de diodes ultraviolettes, de diodes rouges, de diodes bleues, de diodes vertes, de diodes jaunes, de diodes ambres, de diodes orange et de diodes blanches. Il convient également de noter que les LED peuvent être configurées et/ou contrôlées pour générer un rayonnement ayant différentes largeurs de bande (par exemple, largeur complète à mi-maximum, ou FWHM) pour un spectre donné (par exemple, largeur de bande étroite, largeur de bande large) et une variété de longueurs d'onde dominantes au sein d'une catégorisation de couleur générale donnée.

Le terme "source lumineuse" doit être compris comme se référant à une ou plusieurs sources de rayonnement, y compris, mais sans s'y limiter, les sources à base de LED (y compris une ou plusieurs LED telles que définies ci-dessus), les sources incandescentes (par exemple, les lampes à filament, les lampes halogènes), les lampes à incandescence, les lampes à incandescence, etc, lampes à filament, lampes halogènes), sources fluorescentes, sources phosphorescentes, sources à décharge à haute intensité (par exemple, lampes à vapeur de sodium, à vapeur de mercure et à halogénures métalliques), lasers, autres types de sources électroluminescentes, sources pyro-luminescentes (par exemple, flammes), lampes à bougies, etc, flammes), sources luminescentes de bougies (par exemple, manchons de gaz, sources de rayonnement d'arc de carbone), sources photo-luminescentes (par exemple, sources de décharge gazeuse), sources luminescentes de cathode utilisant la satiation électronique, sources galvano-luminescentes, sources cristallo-luminescentes, sources ciné-luminescentes, sources thermo-luminescentes, sources triboluminescentes, sources sonoluminescentes, sources radioluminescentes, et polymères luminescents.

Une source lumineuse donnée peut être configurée pour générer un rayonnement électromagnétique dans le spectre visible, en dehors du spectre visible, ou une combinaison des deux.

Le terme "spectre" doit être compris comme désignant une ou plusieurs fréquences (ou longueurs d'onde) de rayonnement produites par une ou plusieurs sources lumineuses. En conséquence, le terme "spectre" se réfère à des fréquences (ou longueurs d'onde) non seulement dans le domaine visible, mais aussi à des fréquences (ou longueurs d'onde) dans l'infrarouge, l'ultraviolet et d'autres domaines du spectre électromagnétique global. De même, un spectre donné peut avoir une largeur de bande relativement étroite ou une largeur de bande relativement large. Il convient également de noter qu'un spectre donné peut être le résultat d'un mélange de deux ou plusieurs autres spectres (par exemple, mélange de rayonnements émis respectivement par plusieurs sources lumineuses).

Dans le cadre de la présente divulgation, le terme "couleur" est utilisé de manière interchangeable avec le terme "spectre". Toutefois, le terme "couleur" est généralement utilisé pour faire référence à une propriété du rayonnement qui est perceptible par un observateur (bien que cette utilisation ne soit pas destinée à limiter la portée de ce terme). Il convient également de noter que le terme "couleur" peut être utilisé en relation avec la lumière blanche et la lumière non blanche.

Le terme "température de couleur" se réfère essentiellement à une teneur en couleur ou à une nuance particulière (par exemple, rougeâtre, bleuâtre) de la lumière blanche. La température de couleur d'un échantillon de rayonnement donné est généralement caractérisée par la température en degrés Kelvin (K) d'un radiateur à corps noir qui émet essentiellement le même spectre que l'échantillon de rayonnement en question.Les températures de couleur inférieures indiquent généralement une lumière blanche ayant une composante rouge plus importante ou une "sensation de chaleur", tandis que les températures de couleur supérieures indiquent généralement une lumière blanche ayant une composante bleue plus importante ou une "sensation de fraîcheur". À titre d'exemple, le feu a une température de couleur d'environ 1800 degrés Kelvin, une ampoule à incandescence classique a une température de couleur d'environ 2848 degrés Kelvin, la lumière du jour tôt le matin a une température de couleur d'environ 3000 degrés Kelvin, et le ciel couvert de midi a une température de couleur d'environ 10000 degrés Kelvin.

Les termes "dispositif d'éclairage" ou "luminaire" sont utilisés ici de manière interchangeable pour désigner une mise en oeuvre ou un arrangement d'une ou plusieurs unités d'éclairage dans un facteur de forme, un assemblage ou un emballage particulier. Le terme "unité d'éclairage" est utilisé ici pour désigner un appareil comprenant une ou plusieurs sources lumineuses de types identiques ou différents. Une unité d'éclairage donnée peut avoir une variété de dispositions de montage pour la ou les sources lumineuses, de dispositions et de formes de boîtiers et/ou de configurations de connexions électriques et mécaniques. En outre, une unité d'éclairage donnée peut éventuellement être associée à (par exemple, inclure, être couplée à et/ou emballée avec) divers autres composants (par exemple, circuit de contrôle) relatifs au fonctionnement de la ou des sources lumineuses. Une "unité d'éclairage à base de LED" est une unité d'éclairage qui comprend une ou plusieurs sources lumineuses à base de LED telles que décrites ci-dessus, seules ou en combinaison avec d'autres sources lumineuses non basées sur des LED. Une unité d'éclairage "multicanal" désigne une unité d'éclairage à base de LED ou non à base de LED qui comprend au moins deux sources lumineuses configurées pour générer respectivement différents spectres de rayonnement, chaque spectre de source différent pouvant être désigné comme un "sous-canal" de l'unité d'éclairage multicanal.

Le terme "contrôleur" est utilisé ici de manière générale pour décrire divers appareils relatifs au fonctionnement d'une ou de plusieurs sources lumineuses. Un contrôleur peut être mis en oeuvre de nombreuses façons (par exemple, avec du matériel spécialisé) pour exécuter les diverses fonctions décrites dans le présent document. Un "processeur" est un exemple de contrôleur qui utilise un ou plusieurs microprocesseurs pouvant être programmés à l'aide d'un logiciel (par exemple, un microcode) pour exécuter les diverses fonctions décrites dans le présent document. Un contrôleur peut être mis en oeuvre avec ou sans processeur, et peut également être mis en oeuvre comme une combinaison de matériel dédié pour exécuter certaines fonctions et un processeur (par exemple, un ou plusieurs microprocesseurs programmés et des circuits associés) pour exécuter d'autres fonctions. Les exemples de composants de contrôleur qui peuvent être employés dans divers modes de réalisation de la présente divulgation comprennent, sans s'y limiter, des microprocesseurs conventionnels, des circuits intégrés à application spécifique (ASIC).

Dans diverses mises en oeuvre, un processeur ou un contrôleur peut être associé à un ou plusieurs supports de stockage (génériquement appelés ici "mémoire", par exemple, mémoire informatique volatile et non volatile telle que RAM, PROM, EPROM et EEPROM, disquettes, disques compacts, disques optiques, bandes magnétiques, etc.) Dans certaines réalisations, le support de stockage peut être codé avec un ou plusieurs programmes qui, lorsqu'ils sont exécutés par un ou plusieurs processeurs et/ou contrôleurs, exécutent au moins certaines des fonctions décrites dans le présent document. Divers supports de stockage peuvent être fixés dans un processeur ou un contrôleur ou peuvent être transportables, de sorte que le ou les programmes qui y sont stockés peuvent être chargés dans un processeur ou un contrôleur afin de mettre en oeuvre divers aspects de la présente invention.

Les termes logiciel, programme ou programme informatique sont utilisés ici dans un sens générique pour désigner tout type de code informatique (par exemple, logiciel ou microcode) qui peut être utilisé pour programmer un ou plusieurs processeurs ou contrôleurs.

Le terme "interface utilisateur" tel qu'il est utilisé ici fait référence à une interface entre un utilisateur ou un opérateur humain et un ou plusieurs dispositifs qui permet la communication entre l'utilisateur et le(s) dispositif(s). Les exemples d'interfaces utilisateur qui peuvent être utilisées dans diverses mises en oeuvre de la présente divulgation comprennent, sans s'y limiter, des commutateurs, des potentiomètres, des boutons, des cadrans, des curseurs, une souris, un clavier, un pavé numérique, divers types de contrôleurs de jeu (par exemple, des manettes), des billes de piste, des écrans d'affichage, divers types d'interfaces utilisateur graphiques, des écrans tactiles, des microphones et d'autres types de capteurs qui peuvent recevoir une certaine forme de stimulus généré par l'homme et générer un signal en réponse à ce stimulus.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La figure 1 est une vue en perspective d'un système d'éclairage circadien ;
[Fig. 2] La figure 2 est une illustration de deux graphiques correspondant à l'évolution respectivement d'un éclairement lumineux exprimé en lux (2A) et à une température de couleur exprimée en Kelvin (2B) en fonction du temps en minutes pour un premier scénario d'éclairage ;

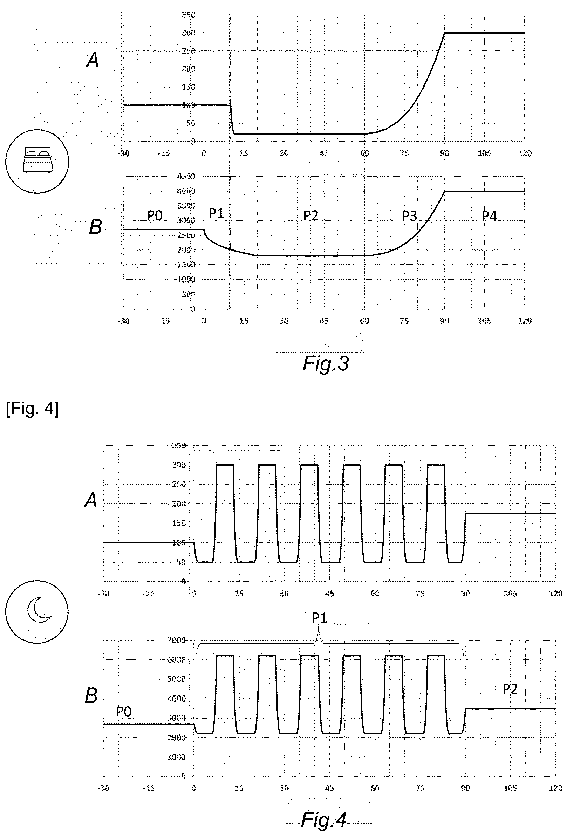

[Fig. 3] La figure 3 est une illustration de deux graphiques correspondant à l'évolution respectivement d'un éclairement lumineux exprimé en lux (3A) et à une température de couleur exprimée en Kelvin (3B) en fonction du temps en minutes pour un deuxième scénario d'éclairage ;
[Fig. 4] La figure 4 est une illustration de deux graphiques correspondant à l'évolution respectivement d'un éclairement lumineux exprimé en lux (4A) et à une température de couleur exprimée en Kelvin (4B) en fonction du temps en minutes pour un troisième scénario d'éclairage.

### Description détaillée de l'invention

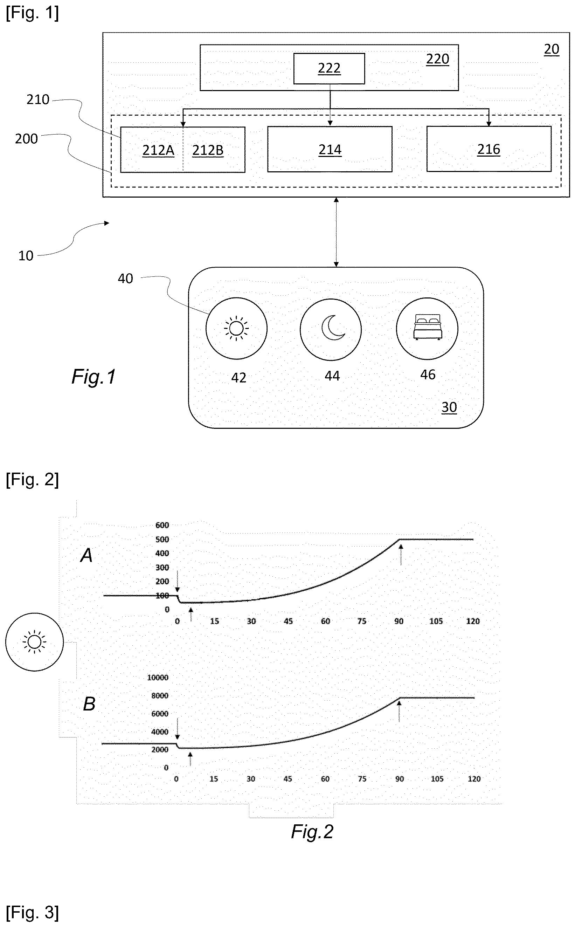

On a représenté sur la figure 1 un système d'éclairage circadien. Ce système est désigné par la référence générale 10.

Comme cela est illustré sur la figure 1, le système 10 comprend un dispositif d'éclairage circadien 20. Le dispositif 20 est un dispositif d'éclairage circadien à source lumineuse accordable configuré pour fournir une pluralité de scénarios d'éclairage de synchronisation d'une horloge biologique d'un individu. En particulier, le dispositif 20 est configuré pour émettre une source de lumière 200 dont la température de couleur peut être modifiée en fonction du temps.

De façon connue en soi, la source de lumière 200 est la résultante du mixage de la lumière générée par une pluralité d'unités 210 d'émission de lumière qui vont être décrites ci-après de façon plus détaillée. Dans le mode de réalisation préféré de l'invention, chaque unité 210 comprend au moins un élément lumineux, par exemple une diode électroluminescente (DEL ou selon l'acronyme anglais LED), et de préférence comprend une pluralité d'éléments lumineux pour chaque unité 210.

Les scénarios d'éclairage peuvent consister de façon plus particulière mais non exclusive en une synchronisation d'une horloge biologique (luminothérapie), une stimulation du corps en vue d'une épreuve sportive ou intellectuelle, un décalage de l'horloge biologique en prévision d'un voyage futur ou une récupération d'un décalage horaire lié à un voyage récent, un protocole d'optimisation de la concentration, de la performance intellectuelle ou physique et des capacités neurocognitives, ou en une aide à l'endormissement.

En particulier, le dispositif 20 comprend un module 220 de commande ou contrôleur de la puissance lumineuse des unités d'éclairage 210 indépendamment l'une de l'autre afin d'obtenir une pondérale spectrale des unités 210, variable en fonction du temps, par la variation de la puissance lumineuse de chacune des unités 210 entre des valeurs maximales et minimales prédéfinies.

En outre, de préférence, le module de commande 220 comprend une unité d'adressage 222 reliée aux unités 210 et chaque unité 210 comprend une adresse unique qui peut être statique ou dynamique.

Le module de commande 220 est prévu par exemple pour transmettre des signaux de pilotage comprenant des informations de pilotage de chaque unité lumineux 210 à l'unité d'adressage 222, par exemple selon un protocole de communication prédéfini connu de l'homme du métier. Chaque signal de pilotage comprend par exemple une structure de données comportant des instructions de pilotage, une adresse et des données utiles. Dans le mode de réalisation préféré de l'invention, le module de commande 220 comprend un processeur pour exécuter des instructions logicielles pour la génération de signaux de pilotage des unités 210. Le module de commande 220 comprend une mémoire pour stocker les instructions logicielles en vue de leur exécution par le processeur.

De préférence, l'unité d'adressage 222 comprend un circuit intégré ou un microcontrôleur relié aux éléments lumineux d'une même unité 210. Par ailleurs, l'unité d'adressage 222 peut comporter un ou plusieurs bus de répartition d'énergie électrique entre chacune des unités d'éléments lumineux 210 afin de permettre un contrôle du flux lumineux de chacune des unités 210.

De préférence, le module de commande 220 comprend encore un processeur pour exécuter des instructions logicielles pour le pilotage des paramètres des éléments lumineux de chaque unité 210. Par exemple, le module de commande 220 comprend une mémoire pour stocker les instructions logicielles en vue de leur exécution par le processeur.

En particulier, le dispositif 20 comprend une première unité d'émission de lumière de couleur blanche 212. Cette première unité 212 émet une lumière dont le spectre lumineux s'étend principalement dans une plage de longueurs d'onde comprise entre 400 nm et 800 nm.

Selon l'invention, la première unité 212 comprend une pluralité d'éléments lumineux émettant chacun un flux lumineux unitaire en direction d'une surface à éclairer.

De préférence, la première unité 212 est formée par une combinaison d'au moins deux groupes 212A, 212B ou sous-canaux d'éléments lumineux, ici des diodes électroluminescentes : un premier groupe ou sous-canal 212A de diodes électroluminescentes émettant une lumière de type blanc chaud avec une température de couleur d'environ 2700K et un deuxième groupe ou sous-canal 212B de diodes électroluminescentes émettant une lumière de type blanc froid avec une température de couleur d'environ 6500K. Ces sous-canaux 212A et 212B peuvent être pilotés de façon indépendante par le module de commande 220.

En outre, selon l'invention, le dispositif 20 comprend une deuxième unité d'émission de lumière 214 de couleur bleue dont le spectre lumineux forme un pic étroit et dont le sommet du pic est compris entre 400 et 500 nm formant une deuxième unité dit de stimulation. Ce pic est de préférence centré autour de 490 nm, avec une marge de plus ou moins 5 nm autour de cette valeur. De préférence, par pic étroit, on entend que la largeur à mi-hauteur du pic étroit est comprise entre 20 et 50 nm. La valeur préférée de 490 nanomètres correspond à un maximum de sensibilité des cellules réceptrices non-visuelles ou glandes à mélanopsine d'un être humain.

Plus spécifiquement, la puissance lumineuse émise par la deuxième unité 214 est toujours inférieure ou égale à 35% de la puissance totale émise par la source lumineuse 200 du dispositif 20. Ceci permet d'optimiser le rendu des couleurs du dispositif 20 sans dénaturer la couleur blanche de la lumière émise par la source 200 tout en permettant une action efficace sur les cellules réceptrices non-visuelles.

En outre, dans un mode de réalisation préféré de l'invention, le dispositif 20 comprend encore une troisième unité 216 dont le spectre lumineux est relativement enrichi de longueurs d'onde supérieures à 560 nm et relativement dépourvu de longueurs d'onde inférieures à 560 nm. De préférence, le spectre lumineux de la troisième unité 216 est formée par un pic centré sur une longueur d'onde supérieure à 560 nm, de préférence centrée sur 590 nm (avec une marge de plus ou moins 5 nm autour de cette valeur), avec une largeur de pic comprise entre 20 et 50 nm. Cette unité 216 permet d'activer le système visuel mais n'a pas d'effet sur les cellules réceptrices non visuelles à mélanopsines d'un être humain. La lumière ambre émise par cette troisième unité 216 permet au corps humain de sécréter naturellement la mélatonine par inhibition des cellules réceptrices à mélanopsines.

De préférence, afin de tenir compte du rythme ultradien de l'être humain, la durée d'un scénario est comprise entre 80 minutes et 100 minutes, de préférence 90 minutes.

Par exemple, le module de commande 220 comprend des moyens de génération des instructions logicielles de pilotage de la source de lumière 200 à partir d'une trame de données de résultats d'un ensemble de données d'évaluation d'un profil d'un utilisateur.

En outre, de préférence, le dispositif 20 comprend de préférence un terminal 30 de commande à distance du module 220 de commande du dispositif 20. De préférence, le dispositif 20 comprend une interface utilisateur 40, supportée par exemple de manière logicielle par le terminal de commande à distance 30.

Le module de commande 220 comprend des moyens de génération des signaux de pilotage des unités d'éclairage 210 en fonction d'une trame de données générée à la suite de la sélection par un opérateur d'un scénario parmi une pluralité de scénarios prédéterminés. Ces instructions logicielles permettent par exemple la génération de signaux de pilotage adressés à l'unité d'adressage 222 puis aux unités 210. L'interface utilisateur 40 est supportée de manière logicielle sur l'équipement électronique externe 30 qui communique avec le dispositif 20. Le module de commande 220 comprend un processeur pour exécuter des instructions logicielles pour la génération de signaux de pilotage des unités 210. En variante, l'interface utilisateur 40 peut être intégrée dans un même boîtier d'emballage du dispositif 20 sans terminal de commande fonctionnement à distance.

Par exemple, les instructions logicielles sont générées à partir de l'interface utilisateur 40 du dispositif 20 permettant à un opérateur de sélectionner les paramètres de réglage de la puissance de chaque unité 210 en fonction d'un profil de l'utilisateur. De préférence, l'interface utilisateur 40 est supportée de manière logicielle sur une tablette 30. La tablette 30 communique par exemple avec le module de commande 220 par une liaison filaire ou sans fil (par exemple par Bluetooth^{®} ou par WiFi).

Dans un mode de réalisation préféré de l'invention, le dispositif 20 comprend un module de recommandation configuré pour générer un niveau de recommandation pour chaque scénario parmi la pluralité de scénarios prédéterminés avec un créneau horaire d'application de chacun des scénarios.

De préférence ledit module de recommandation comprend un moyen de saisie pour recueillir des données relatives à un utilisateur et un moyen de détermination d'un scénario à partir d'une combinaison des données saisies et d'une information d'un créneau horaire local actuel. Les moyens de détermination comprennent par exemple une table de correspondance prenant en entrée d'une part les données utilisateurs et les créneaux horaires d'une journée et fournissant en sortie des niveaux de recommandation pour les trois scénarios (ou plus).

Les données relatives à l'utilisateur comprennent par exemple une liste de paramètres choisis parmi un chronotype (matinal, vespéral ou mixte), une heure de réveil et/ou une heure de coucher, un décalage horaire lié à la traversée de plusieurs fuseaux horaires vers l'Est ou vers l'Ouest et un déficit de sommeil.

Dans un mode de réalisation préféré de l'invention, l'interface utilisateur 40 comprend une pluralité d'icônes représentant chacun un scénario. Dans l'exemple illustré sur la figure 1, l'interface utilisateur 40 comprend des première 42, deuxième 44 et troisième 46 icônes correspondant respectivement à un scénario de réveil, un scénario de relaxation dynamique et un scénario de récupération qui vont être détaillés ci-après.

Le module de recommandation comprend par exemple des moyens visuels d'affichage différencié des icônes entre elles, par exemple une mise en surbrillance ou un affichage d'un score de recommandation sur une échelle de 0% à 100% associé à chaque icône.

On va maintenant décrire trois scénarios de synchronisation d'une horloge biologique d'un utilisateur.

De préférence, quel que soit le scénario, initialement, la source de lumière 200 est accordée spectralement de préférence à une lumière blanche chaud d'accueil : par exemple, éclairement de 100 lux et température de couleur blanc chaud de 2700K.

Dans un premier scénario de réveil, la variation de la température de couleur est représentée sur la courbe 2B et la variation de l'éclairement est représentée sur le courbe 2A. Au cours d'une première période T1, les unités 212A et 216 respectivement de lumière blanc chaud et de lumière ambre sont activées de telle sorte que la température de couleur décroît jusqu'à atteindre une température de couleur de 2100K. Pendant cette période T1, la lumière émise par la source 200 tend à reproduire le lever du soleil ou l'aube ce qui permet à l'individu de synchroniser son horloge biologique sur un début de matinée. Puis, au cours d'une période T2, l'unité de lumière ambre 216 est désactivée et le sous-canal de l'unité de lumière blanche 212B de 6500K est activée de sorte que la température de couleur de la source 200 croît d'une température de 2700K à 6500K.

En outre, la puissance de l'unité de lumière cyan 214 est progressivement augmentée d'une valeur sensiblement nulle jusqu'à atteindre une valeur maximale inférieure à 35% de la puissance totale de la source de lumière 200 dans une période T3. De préférence, le module de commande 220 est configuré pour faire varier progressivement la puissance lumineuse de la deuxième unité 214 d'une puissance nulle jusqu'à une puissance maximale correspondant à au plus 35% de la puissance totale.

Dans un scénario de relaxation dynamique, la variation de la température de couleur est représentée sur la courbe 4B et la variation de l'éclairement correspondant est représenté sur la courbe 4A. Dans ce scénario, le module de commande 220 est configuré pour faire varier la puissance lumineuse de la deuxième unité 214 entre une valeur nulle et une valeur maximale de puissance selon un profil de trains d'impulsions. Par exemple, pour une durée de traitement de 90 minutes, le profil de trains d'impulsions comprend entre quatre et huit impulsions, ici cinq impulsions. Les impulsions oscillent entre une température de couleur d'environ 2100 Kelvins (activation de l'unité ambre 216 et désactivation de l'unité de la lumière cyan 214) et une température de couleur d'environ 6000 Kelvins (activation de l'unité de la lumière cyan 214 et désactivation de la lumière de l'unité ambre 216). L'unité de lumière blanche 212 est de préférence maintenue activée au cours de ce scénario. En particulier, la valeur de puissance maximale de la lumière émise par l'unité 214 est toujours inférieure à 35% de la puissance totale de la source lumineuse 200. De préférence, la valeur de la puissance maximale de la lumière 214 correspond à 30% de la puissance totale de la source 200.

Enfin, dans un troisième scénario dit de récupération, la variation de la température de couleur est représentée sur la courbe 3B et la variation de l'éclairement correspondant en lux est représenté sur la courbe 3A. De préférence, dans ce scénario, le module de commande 220 est configuré pour faire varier la puissance lumineuse de la première unité 212 jusqu'à une puissance sensiblement nulle pendant une période T1 et pour maintenir uniquement la puissance lumineuse de la troisième unité 216 à une valeur non nulle pendant une période T2. Pendant une période T3, les trois unités 212 à 216 sont progressivement activées pour augmenter progressivement la température de couleur de 1800K à 4000K. L'utilisation d'une lumière de plus en plus ambrée pendant la période T2 permet d'accompagner le corps à la sécrétion de mélatonine, ce qui va favoriser la relaxation.

On va maintenant décrire les principaux aspects de fonctionnement du dispositif d'éclairage 20, en relation avec un utilisateur souhaitant bénéficier d'une séance de luminothérapie en rapport à avec son activité passée ou future.

Dans un premier temps, un opérateur procède à une récupération des données de l'utilisateur : chronotype (matinal, vespéral ou mixte), temps moyen de sommeil, déficit de sommeil et/ou événement passé ou à venir de décalage horaire. Le chronotype de l'utilisateur pourra être déterminé par exemple selon l'heure privilégiée de début de sommeil et l'heure privilégiée de réveil de l'utilisateur. Concernant l'événement de décalage horaire, l'opérateur indique par exemple un nombre de fuseaux horaires de décalage vers l'Est ou vers l'Ouest traversés par l'utilisateur. L'opérateur saisit ces données via par exemple l'interface utilisateur 40.

L'opérateur indique également par l'intermédiaire de l'interface utilisateur 40 le créneau horaire actuel local (par exemple 9h-11h, 11h-13h, 13h-15h, 15h-17h, ou 17h-19h). Ce créneau horaire local actuel peut également être calculé automatiquement en récupérant une heure actuelle locale d'une horloge interne du dispositif 20. Le module de recommandation calcule alors pour chaque scénario un score de recommandation qui s'affiche sur l'interface utilisateur 40 avec un créneau horaire recommandé pour chaque scénario.

L'unité de couleur cyan 214 du dispositif 20 est activée dans chaque scénario d'éclairage et permet d'accentuer artificiellement la stimulation des voies non visuelles du corps humain ce qui impacte directement la sécrétion d'hormones.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Dispositif (20) d'éclairage circadien comprenant une source lumineuse (200) accordable pour fournir une pluralité de scénarios d'éclairage prédéterminés, **caractérisé en ce que** la source (200) comprend une première unité (212) de lumière blanche dont le spectre lumineux s'étend principalement dans une plage de longueurs d'onde comprise entre 400 nm et 800 nm et une deuxième unité (214) de lumière bleue dont le spectre lumineux forme un pic étroit dont le sommet est compris entre 400 et 500 nm et **en ce que** le dispositif (20) comprend un module de commande (220) de la puissance lumineuse des deux unités (210 ; 212, 214) indépendamment l'une de l'autre afin d'obtenir un ratio de mixage spectral des deux unités, variable en fonction du temps, par la variation de la puissance lumineuse de chacune des unités (210 ; 212, 214) entre des valeurs maximales et minimales, la puissance lumineuse émise par la deuxième unité (214) étant toujours inférieure ou égale à 35% de la puissance totale émise par la source lumineuse (200).

2. Dispositif (20) selon la revendication précédente, dans lequel dans un scénario de stimulation, le module de commande (220) est configuré pour faire varier progressivement la puissance lumineuse de la deuxième unité (214) d'une puissance nulle jusqu'à une puissance maximale correspondant à au plus 35% de la puissance totale de la source lumineuse (200).

3. Dispositif (20) selon l'une quelconque des revendications précédentes, dans lequel la première unité (212) de lumière blanche comprend un premier sous-canal (212A) de lumière blanc chaud avec une température de couleur autour de 6500 Kelvin et un deuxième sous-canal (212B) de lumière blanc froid avec une température de couleur autour de 2700 Kelvin.

4. Dispositif (20) selon l'une quelconque des revendications précédentes, dans lequel le module de commande (220) est configurée pour faire varier la puissance lumineuse de la deuxième unité (214) entre une valeur nulle et une valeur maximale selon un profil de trains d'impulsions, par exemple de période de répétition comprise entre 12 minutes et 18 minutes.

5. Dispositif (20) selon la revendication précédente, dans lequel la source (200) comprend une troisième unité (216) dont le spectre lumineux est relativement enrichi de longueurs d'onde supérieures à 560nm et relativement dépourvu de longueurs d'onde inférieures à 560 nm.

6. Dispositif (20) selon la revendication précédente, dans lequel, dans un scénario de récupération, le module de commande (220) est configuré pour faire varier la puissance lumineuse de la première unité (210) jusqu'à une puissance sensiblement nulle et pour maintenir uniquement la puissance lumineuse de la troisième unité (216) à une valeur non nulle.

7. Dispositif (20) selon l'une quelconque des revendications précédentes, dans lequel la durée d'un scénario est comprise entre 80 minutes et 100 minutes, de préférence 90 minutes.

8. Dispositif (20) selon l'une quelconque des revendications précédentes, dans lequel, le dispositif (20) comprenant une interface utilisateur (40), le module de commande (220) comprend un moyen de génération des signaux de pilotage des unités d'éclairage (210 ; 212, 214, 216) en fonction d'une trame de données générée consécutivement à la sélection par un opérateur d'un scénario parmi une pluralité de scénarios prédéterminés.

9. Dispositif (20) selon l'une quelconque des revendications précédentes, comprenant un module de recommandation configuré pour générer un niveau de recommandation pour chaque scénario d'éclairage et un créneau horaire recommandé pour appliquer chaque scénario.

10. Dispositif (20) selon la revendication précédente, dans lequel ledit module de recommandation comprend un moyen de saisie pour recueillir des données relatives à un utilisateur et un moyen de détermination du niveau de recommandation pour chaque scénario à partir d'une combinaison des données saisies et d'un créneau horaire actuel local.

11. Dispositif (20) selon la revendication précédente, dans lequel les données relatives à l'utilisateur comprennent une liste de paramètres choisis parmi un chronotype, une heure de réveil et/ou une heure de coucher et un décalage horaire.

12. Dispositif (20) selon l'une quelconque des revendications précédentes, comprenant une interface utilisateur (40) supportée de manière logicielle sur un équipement électronique (30) qui communique à distance avec le module de commande (220).
